# EUROPEAN PATENT APPLICATION

(11) **EP 1 843 401 A1**
(43) Date of publication of application: **10.10.2007**
(21) Application number: 06711897.6
(22) Date of filing: 18.01.2006
(51) Int. Cl.: H01L 33/00, A61L 9/00, A61L 9/20, B01D 39/14, B01D 53/86, B01J 19/12, B01J 35/02, C02F 1/32, C02F 1/72

(54) **SURFACE EMITTING DEVICE**

(30) Priority: 26.01.2005 JP 2005017842
(71) Applicant: Sumitomo Electric Industries, Ltd., Osaka-shi, Osaka 541-0041 (JP)
(72) Inventor: KAWAI, Chihiro, 1-1 Koyakita 1-chome, Itami-shi, Hyogo (JP); INOUE, Ryuichi, 1-1 Koyakita 1-chome, Itami-shi, Hyogo (JP)
(74) Representative: HOFFMANN EITLE
(86) International application number: PCT/JP2006/300628
(87) International publication number: WO 2006/080216

(57) **Abstract**

The present invention provides a surface light emitting device which utilizes special features of a back light, and emits ultraviolet light mainly for the purpose of exciting a photocatalyst. Furthermore, the present invention provides a compact thin surface emitting device which utilizes the special features of the back light, and which can also function as a filter that allows the passage of a fluid. The present invention provides a surface emitting device which includes a light source; and a light guide plate with the light source disposed on a side surface thereof, wherein at least one of the front surface and rear surface of the light guide plate is a light emitting surface for emitting light from the light source; surfaces other than the light emitting surface of the light guide plate and the side surface on which the light source is disposed are formed as light shielding surfaces; and the emitted light has a peak wavelength of 388 nm or less, or wherein the light guide plate is formed as a porous body. The surface emitting device can be one in which the light guide plate has a plurality of through-holes, and the surface in which the through-holes are formed is a light emitting surface.

## Description

### TECHNICAL FIELD

The present invention relates to a device that can convert a point light source or linear light source having a function for radiating visible light or ultraviolet light into a planar light source. Furthermore, the present invention relates to a surface emitting device which has the function of directly killing microbes or decomposing organic matter using visible light or ultraviolet light irradiated following conversion into a planar light source, or of exciting a photocatalyst via irradiated light to decompose harmful matter or kill microbes. The present invention further relates to a filter using this surface emitting device.

### BACKGROUND ART

In air cleaners or other conventional harmful matter decomposing devices utilizing photocatalysts, a material having a photocatalytic function such as TiO₂ or the like is supported on a porous body to form a photocatalyst sheet, and the photocatalyst is excited via the irradiation of this sheet with ultraviolet light radiated from a linear light source or point light source such as a mercury lamp, light emitting diode or the like.
However, in the case of a point light source or linear light source, the overall photocatalyst sheet cannot be irradiated with light unless the light source is installed at an appropriate distance from the photocatalyst sheet. Accordingly, the overall size of the apparatus is increased. Furthermore, the following problem also arises: in the case of a liquid with a high turbidity, the light from the light source is attenuated by the time this light reaches the photocatalyst.

On the other hand, a method in which the photocatalyst is excited using a surface emitting body as a light source can also be considered (See Patent Document 1). In such a method, the photocatalyst is excited using as a light source an inorganic EL light emitting sheet which emits short-wavelength visible light to ultraviolet light. For example, the sheet is folded double, and harmful components present in a fluid positioned between the folds of the sheet are decomposed and removed by the photocatalytic action. In this structure, however, it is necessary to increase the number of laminated layers so that numerous flow channels are created in order to treat large amounts of fluid, so that the apparatus itself is extremely large.

In contrast to these methods, a ceramic filter has been invented in which the light emitting body and the electrode itself have a porous structure, and the filter has a function in which ultraviolet light is emitted from the porous body when the fluid passes through this structure, so that a photocatalyst supported in this porous body decomposes organic matter or kills bacteria, viruses or the like (see Patent Document 2). The light emitting body is obtained by sintering semiconductor particles to a certain degree.

However, the following problems are encountered with this method:
(1) A high degree of technology is required for controlling the pore diameter and porosity of the porous light emitting layer. In particular, in cases where the filter is used in an air cleaner or the like in which a high permeability is required, the pore diameter and porosity must be set at large values, and semiconductor particles having a large particle size must be used. However, when the particle size is increased, the sinterability drops. Furthermore, in methods in which a powder is sintered, it is difficult to obtain a light emitting layer that has a high porosity.
(2) Since electrodes are formed on the surface of the porous body by sputtering or vapor deposition, the cost is high.
(3) In cases where a liquid, especially a liquid which has a high conductivity, is passed through the interior of the porous light emitting layer, there may be instances in which an electric field cannot be efficiently applied unless the particles forming the porous light emitting layer and electrodes or the like are completely insulated. However, such an insulation treatment requires a high degree of technology. In particular, an even higher degree of technology is required when the constituent particle size is small, so that the cost is increased.
(4) In inorganic EL devices, light is generally emitted by applying an alternating-current electric field of 100 V or greater with a frequency of several hundred to several thousand Hertz. An inverter that applies such an electric field is separately required, so that extra space is required.

Liquid crystal display backlighting light sources are available as products which convert LEDs or mercury lamps having high light emission intensity into a surface light source. In such light sources, the light of the light source is temporarily guided onto a light guide plate on a flat surface, and further extracted in the direction perpendicular to the surface of the light guide plate. The extracted light passes through the liquid crystal and forms a display. The light source is disposed on the bottom or side surface of the light guide plate; in most cases, the light source is disposed on the side surface in order to obtain compact and thin backlighting.

However, backlighting used in the past has been limited to liquid crystal panels, and has been restricted to displays emitting visible light centering on white light. In particular, since liquid crystals are degraded by ultraviolet light, such displays have been limited as much as possible to displays that do not emit ultraviolet light (see Patent Document 3).
Patent Document 1: Japanese Laid-Open Patent Application Publication No. 2003-200043
Patent Document 2: PCT/WO 2004/006969
Patent Document 3: Japanese Laid-Open Patent Application Publication No. 2002-133930

### DISCLOSURE OF THE INVENTION

### PROBLEMS WHICH THE INVENTION IS INTENDED TO SOLVE

The present invention provides a surface emitting device which takes advantage of the special features of the back light, and which can emit ultraviolet light mainly for the purpose of exciting a photocatalyst. Furthermore, the present invention provides a compact thin surface emitting device which takes advantage of the special features of the back light, and which also functions as a filter that allows the passage of a fluid.

### MEANS USED TO SOLVE THE ABOVE-MENTIONED PROBLEMS

The inventor invented a thin light emitting device having a unique structure as a method for solving the problems described above. Specifically, the present invention has the following constitution:
(1) A surface emitting device, which has a light source; and a light guide plate with the light source disposed on a side surface thereof, wherein at least one of the front surface and rear surface of the light guide plate is a light emitting surface for emitting light from the light source; surfaces other than the light emitting surface of the light guide plate and the side surface on which the light source is disposed are formed as light shielding surfaces; and the emitted light has a peak wavelength of 388 nm or less.

(2) A surface emitting device, which has a light source; and a light guide plate with the light source disposed on a side surface thereof, wherein at least one of front and rear surfaces of the light guide plate is a light emitting surface for emitting light from the light source; surfaces other than the light emitting surface of the light guide plate and the side surface on which the light source is disposed are formed as light shielding surfaces; and the light guide plate is formed as a porous body.
(3) The surface emitting device according to the second aspect, wherein the light guide plate formed as a porous body has through-holes for communicating between the front surface and rear surface.

(4) The surface emitting device according to any one of the first through third aspects, wherein the light guide plate has a flat plate shape or curved surface shape.
(5) The surface emitting device according to the third aspect, wherein the surface emitting device has a plurality of light guide plates of predetermined shape disposed with a predetermined gap left between the plates, the gaps constituting through-holes.

(6) The surface emitting device according to any one of the second through fifth aspects, wherein the surface emitting device emits light having a peak wavelength of 540 nm or less.
(7) The surface emitting device according to the sixth aspect, wherein the surface emitting device emits light having a peak wavelength of 420 nm or less.
(8) The surface emitting device according to the seventh aspect, wherein the surface emitting device emits light having a peak wavelength of 388 nm or less.
(9) The surface emitting device according to any one of the first through eighth aspects, wherein the surface emitting device emits light having a peak wavelength within a range of 355 to 375 nm.

(10) The surface emitting device according to any one of the first through ninth aspects, wherein the light source is an LED.
(11) The surface emitting device according to any one of the first through ninth aspects, wherein the light source is an inorganic EL.
(12) The surface emitting device according to any one of the first through ninth aspects, wherein the light source is a cold cathode fluorescent lamp.

(13) The surface emitting device according to any one of the first through twelfth aspects, wherein a photocatalyst or a porous body supporting a photocatalyst is disposed on the light emitting surface of the light guide plate.
(14) The light emitting device according to the thirteenth aspect, wherein a distance between the light guide plate and the photocatalyst or porous body supporting this photocatalyst is zero.
(15) The surface emitting device according to the thirteenth aspect, wherein at least one of a diffusion sheet and a focusing sheet is disposed between the light emitting surface of the light guide plate and the photocatalyst or porous body supporting the photocatalyst.
(16) The surface emitting device according to any one of the thirteenth through fifteenth aspects, wherein the photocatalyst is a rutile titanium oxide.

(17) A surface emitting device, which has a light source; and a light guide plate with the light source disposed on a side surface thereof, wherein a plurality of through-holes that communicate between a front and rear surface are provided so that a fluid can pass through this light guide plate; and the surface in which these through-holes are formed is a light emitting surface for emitting light from the light source.
(18) The surface emitting device according to the seventeenth aspect, wherein surfaces other than the light emitting surface and the side surface on which the light source is disposed are formed as light shielding surfaces.

(19) A filter in which the surface emitting device according to any one of the second through eighteenth aspects is used.
(20) An air cleaner or air conditioner filter in which the filter according to the nineteenth aspect is used.
(21) A liquid cleaning filter in which the filter according to the nineteenth aspect is used.

### EFFECT OF THE INVENTION

The present invention is a device which has a light source and a light guide plate with this light source disposed on a side surface thereof, and which can convert a linear or point light source such as a mercury lamp, LED or the like, and especially an LED which is a point light source, into a planar light source. By selecting the wavelength of the light source, it is possible to form a compact thin surface emitting device which has various functions. Furthermore, by forming the light guide plate with a porous structure, it is possible to obtain a device which also functions as a filter that can allow the passage of a fluid through the interior, and which can be utilized as the filter of an air cleaner or the like by combining this device with a photocatalyst.

### BRIEF DESCRIPTION OF THE DRAWINGS

[FIG. 1A] A side view showing one example of the structure of the surface emitting device of the present invention in which through-holes are formed.
[FIG. 1B] A perspective view showing one example of the structure of the surface emitting device of the present invention in which through-holes are formed.
[FIG. 2] A diagram showing another example of the structure of the surface emitting device of the present invention in which through-holes are formed.
[FIG. 3A] A diagram showing another example of the structure of the surface emitting device of the present invention in which through-holes are formed.
[FIG. 3B] A diagram showing another example of the structure of the surface emitting device of the present invention in which through-holes are formed.
[FIG. 3C] A diagram showing another example of the structure of the surface emitting device of the present invention in which through-holes are formed.
[FIG. 4] A diagram showing one example of the structure of the surface emitting device of the present invention.
[FIG. 5] A diagram showing the structure of the light guide plate of the present invention.
[FIG. 6] A schematic diagram of the apparatus used for photocatalyst evaluation in Working Example 1.
[FIG. 7] A diagram showing the shape of the light guide plate used in Working Example 2.
[FIG. 8A] A schematic diagram of the apparatus used for photocatalyst evaluation in Working Example 2.
[FIG. 8B] A schematic diagram of the apparatus used for photocatalyst evaluation in Working Example 2.

### BEST MODE OF CARRYING OUT THE INVENTION

The surface emitting device of the present invention will be described in detail.
The surface emitting device of the present invention has a light source; and a light guide plate with the light source disposed on a side surface thereof, wherein at least one of the front surface and rear surface of the light guide plate is a light emitting surface for emitting light from the light source; surfaces other than the light emitting surface of the light guide plate and the side surface on which the light source is disposed are formed as light shielding surfaces; and the emitted light has a peak wavelength of 388 nm or less.
Furthermore, the surface emitting device of the present invention is a surface emitting device which has a light source; and a light guide plate with the light source disposed on a side surface thereof, wherein at least one of front and rear surface of the light guide plate is a light emitting surface for emitting light from the light source; surfaces other than the light emitting surface of the light guide plate and the side surface on which the light source is disposed are formed as light shielding surfaces; and the light guide plate is formed as a porous body.

A portion of the light that is emitted from the light source disposed on the side surface of the light guide plate is reflected by an indented and projecting pattern formed on the front surface or rear surface of the light guide plate, or both, while the light passes through the interior of the light guide plate, and this light is emitted to the outside in a direction that is substantially perpendicular to the surface of the light guide plate.
In cases where the emission wavelength of the light emitted from the light guide plate is 388 nm or less, the capacity for killing microbes and decomposing organic matter is increased. Furthermore, ultraviolet light having a wavelength near 365 nm can be used to cure ultraviolet-curable resins. Moreover, by disposing a photocatalyst on the light emitting surface, it is possible to obtain a surface emitting device which has the function of decomposing harmful matter and killing microbes by exciting this photocatalyst with ultraviolet light.

The device can also be caused to function as a type of filter if a plurality of through-holes that allow the passage of a fluid are formed so that these through-holes communicate between the front surface and rear surface of the light guide plate, and a fluid is circulated so that this fluid passes through the through-holes.
For example, if porous layers (photocatalyst sheets) which have a photocatalyst are superimposed on both surfaces of a surface emitting device of the type that emits light from both surfaces (front surface and rear surface), the contaminated fluid passes through in a direction perpendicular to the surfaces of the photocatalyst sheets. Accordingly, the light and photocatalyst can be constantly maintained in contact with good efficiency. The light that is emitted from the front surface and rear surface of the light guide plate is not necessarily emitted only in the direction perpendicular to the surface of the light guide plate, but spreads at a certain angle. It is thus possible not only to excite the photocatalyst that is positioned directly above the light emitting surface in which no through-holes are formed, but also the photocatalyst that is positioned directly above the through-holes. The fluid makes optimally efficient contact with the photocatalyst that is positioned directly above the through-holes. It is accordingly desirable for the through-holes to be smaller in size, with a diameter of approximately 5 mm being the upper limit for a high effect. If the through-holes are too small, the pressure loss during the passage of the fluid through the through-holes is increased. A lower limit of several tens of µm allows a strong effect to be obtained, regardless of the shape of the through-hole.
When the fluid passes through, it is also possible to obtain a heat radiating effect for dissipating the heat that is generated in cases where the light guide plate is heated.

Meanwhile, in the present invention, the visible light or ultraviolet light that is emitted from the light guide plate can also be concentrated in the through-holes.
In the light guide plate, the surfaces other than the side surface on which the light source is disposed and the surface constituting the surface of the through-holes are formed as light shielding surfaces. Accordingly, the through-holes can be used as light emitting surfaces by emitting light from the light source from the surface constituting the surface of the through-holes.
In a device in which a photocatalyst is disposed on the front surface and rear surface of the light guide plate, it is evident that the passage of the fluid is mainly through the areas of the photocatalyst directly above the flow channels (through-holes). It is possible to realize the most highly efficient photocatalytic performance by concentrating the light in these areas. Furthermore, it is not necessary for the size of the lattice forming the flow channels to be 5 mm or less. This is because the light is concentrated in the flow channels.

Such surface emitting devices can be made extremely thin by disposing the light source on the side surface of the light guide plate, and disposing the photocatalyst on the light emitting surface of the light guide plate. The catalytic reaction can be caused to proceed with good efficiency. For example, if the device is installed as an air cleaner or air conditioner filter, a photocatalytic performance that is comparable to or greater than that obtained in cases where the photocatalyst is directly irradiated with a conventional mercury lamp can be realized with a great reduction in the area occupied by the apparatus. In particular, since the light emitting surface constituting the light source for the photocatalyst is located in close proximity to the photocatalyst, a catalytic reaction can be caused to proceed with good efficiency even in the case of a contaminated fluid showing extreme absorption of ultraviolet light, and incapable of being treated using an external light source such as a mercury lamp or the like.

The photocatalyst sheet is a sheet in which a powdered photocatalyst such as titanium oxide is supported on a resin or other porous body having a high porosity. As the sheet thickness is increased, it becomes difficult for light to pass through; accordingly, in cases where sheets of the same thickness are used, it is desirable to cut the thickness in half, and to irradiate the respective photocatalysts with light.
Accordingly, in the present invention, when indentations and projections are formed in both surfaces (front surface and rear surface) of the light guide plate, light is emitted from both surfaces; accordingly, by installing photocatalyst sheets on both surfaces of the light guide plate, it is possible to realize a higher photocatalytic performance than in cases where a single surface is used.

In cases where the distance between the light guide plate and the photocatalyst or porous body supporting a photocatalyst is zero, the photocatalyst can be excited most efficiently. In cases where the photocatalyst sheet is excited using an external light source such as a mercury lamp or the like, if the light is directed onto a certain area, it is necessary to dispose the external light source at a certain distance from the photocatalyst sheet. In this case, a portion of the light that is emitted from the light source is reflected by the photocatalyst sheet, so that the photocatalyst cannot be excited, thus resulting in an energy loss. In the present invention, on the other hand, as a result of the photocatalyst being disposed directly above or directly beneath the light emitting surface of the light guide plate, all of the light that is emitted from the light guide plate can be closed inside the photocatalyst sheet, so that the photocatalyst can be excited.

In the light guide plate of the present invention, which has through-holes, it is also possible to dispose a plurality of light guide plates at a specified gap spacing, so that the gaps form flow channels (through-holes), instead of forming through-holes in a light guide plate.
A structure that is easy to manufacture is one in which rectangular light guide plates are disposed side by side with gaps at a specified spacing.

In the present invention, there are also cases in which a diffusion sheet or focusing sheet, or both, are disposed on the light emitting surface of the light guide plate. The diffusion sheet has the effect of spreading the light emitted to the outside from the light guide plate even more uniformly in the planar direction, and can uniformly irradiate the photocatalyst with light. The focusing sheet is used to reduce the emission angle of the light emitted from the light guide plate; for example, this has an effect in cases where the light guide plate and photocatalyst are disposed at a distance from each other, and the areas of the two elements are about the same. This is not necessary in cases where the photocatalyst sheet is disposed directly above the light emitting surface of the light guide plate or the like.

The diffusion sheet is formed from a transparent resin such as an acrylic resin (PMMA), polycarbonate (PC), cycloolefin polymer or the like. The diffusion sheet has surfaces with microscopic indentations and projections, and acts to spread the light so that the light source has a broad light cone.

In cases where a visible light response photocatalyst is used as the photocatalyst, it is desirable that the surface emitting device emit light having a peak wavelength of 540 nm or less. If this wavelength is exceeded, the photocatalytic performance cannot be sufficiently realized. If ultraviolet light having a peak wavelength of 420 nm or less is emitted, a rutile titanium oxide having a high photocatalytic activity can be used; accordingly, such ultraviolet light is desirable. It is even more desirable that the peak wavelength of the emission spectrum be 388 nm or less. At this wavelength or less, an anatase titanium oxide which has a high photocatalytic activity like rutile titanium oxide, and which is inexpensive, can be most efficiently excited. Furthermore, when the wavelength of the ultraviolet light is 365 nm, the application range of the present invention is further expanded. Ultraviolet light having a wavelength of 365 nm is most commonly used as ultraviolet light for the curing of ultraviolet-curable resins, and is also the wavelength preferred by insects, so that this light can also be used in devices that collect insects. These effects are high when the emission peak wavelength of the surface emitting device is in the range of 355 to 375 nm. When the wavelength of the ultraviolet light is 300 nm or less, bacteria, viruses and the like can be killed even if a photocatalyst is not used. Most desirable is a wavelength of 240 to 280 nm.

A CCFL (cold cathode fluorescent lamp) or the like can be used as the light source; this lamp has a rectilinear shape, L shape, C shape or the like, and the light is directly incident on the interior of the light guide plate from the incident part of the light guide plate. Furthermore, the light source is not limited to a linear CCFL; an array-form light source in which semiconductor light emitting elements are lined up side by side may also be used as a linear light source. Furthermore, the light source may also be constructed from (for example) an LED, laser or the like using semiconductor elements. A mercury lamp such as a cold cathode fluorescent lamp or the like may also be used as the light source; however, it is desirable to use a green, blue or ultraviolet LED in order to reduce the space occupied by the light source. When the point light source or linear light source described above is used as a light source, this can be converted into a surface light source. Furthermore, a direct-current inorganic EL surface emitting body which realizes a high light intensity may also be used. In cases where a surface emitting body is used as the light source, all of the area on both sides of the light guide plate can be irradiated with light; accordingly, the advantage of a high uniformity of the light intensity of the light emitting surface can be obtained.
Furthermore, depending on the shape of the light source, there are cases in which the light source must be disposed at some distance from the side surface; however, it is desirable to dispose the light source as close as possible to the side surface of the light guide plate, since this reduces the space that is occupied by the light source.

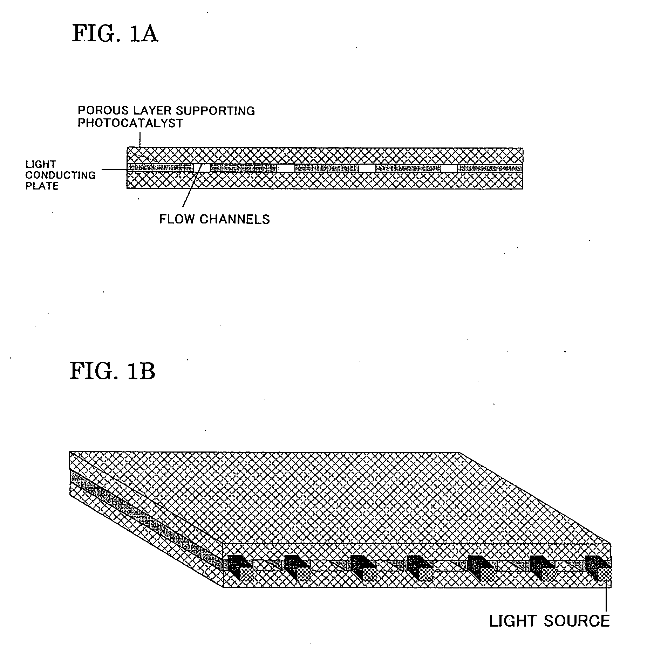

One example of the specific structure (first structure) of the surface emitting device of the present invention in which through-holes are formed is shown in FIG. 1. In FIG. 1, A is a side view, and B is a perspective view.
In the surface emitting device shown in FIG. 1, the front surface and rear surface are light emitting surfaces. A plurality of rectangular light guide plates in which the surfaces other than the light emitting surfaces and the side surface on which the light source is disposed are formed as light shielding surfaces are disposed side by side with a specified gap spacing being left, and porous layers which have a photocatalyst (photocatalyst sheets) are disposed on the light emitting surfaces of the light guide plates.
The fluid that is being treated flows in from the surface of one light emitting surface, passes through the gaps between the light guide plates, and is discharged via the porous layer on the opposite side. The visible light or ultraviolet light emitted from the light guide plates is directed onto the photocatalyst sheets, and can excite the photocatalyst. The fluid that flows through the porous layer on one side passes through the gaps between the light guide plates, and is finally discharged as a clean fluid via the porous layer on the other side. The permeation rate of the fluid improves along with an increase in the ratio of the area occupied by the gaps between the light guide plates to the overall area of the front surface (upper surface) or rear surface (undersurface) of the light guide plates ("flow channel area ratio"). Conversely, as the area of the light emitting portions drops, the porous bodies that have a photocatalyst are not uniformly irradiated with light, or the brightness drops. As a rule, the flow channel area ratio is desirably 30% to 70% of the total area.

Furthermore, for example, the surface emitting device in which light from the light source is emitted from the side surface in which through-holes are formed can be obtained by forming the front surfaces and rear surfaces of the rectangular light guide plates lined up side by side with fixed gaps left in between as described in FIG. 1, and the side surfaces facing the light source, as light shielding surfaces. The front and rear surfaces can be formed as light shielding surfaces by a method such as forming indentations and projections in the front and rear surfaces of the light guide plates, or forming totally reflective plates on these front and rear surfaces. Furthermore, the side surfaces facing the light source can be formed as light shielding surfaces by forming metal coatings or the like on these surfaces.

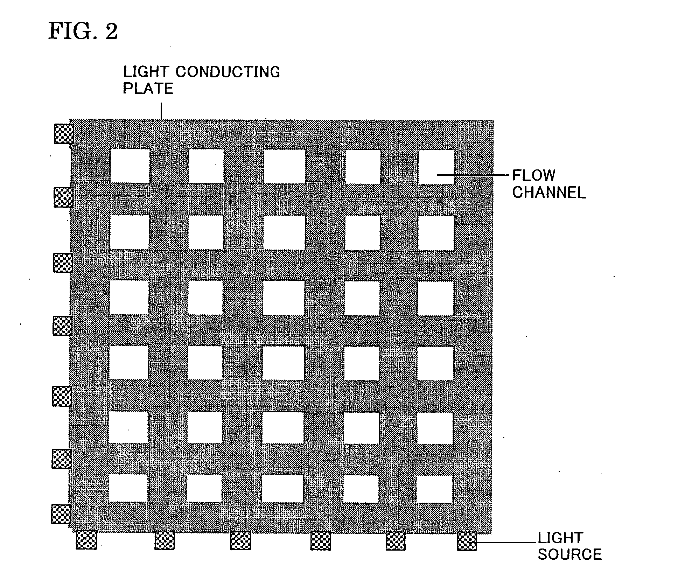

As is shown in FIG. 2, the second structure of the surface emitting device of the present invention in which through-holes are formed is a structure in which through-holes are formed in the form of a lattice. This structure is easier to manufacture than the first structure. Furthermore, the through-holes may be formed as long, slender slit-shaped through-holes. (In FIG. 2, the photocatalyst is omitted.)

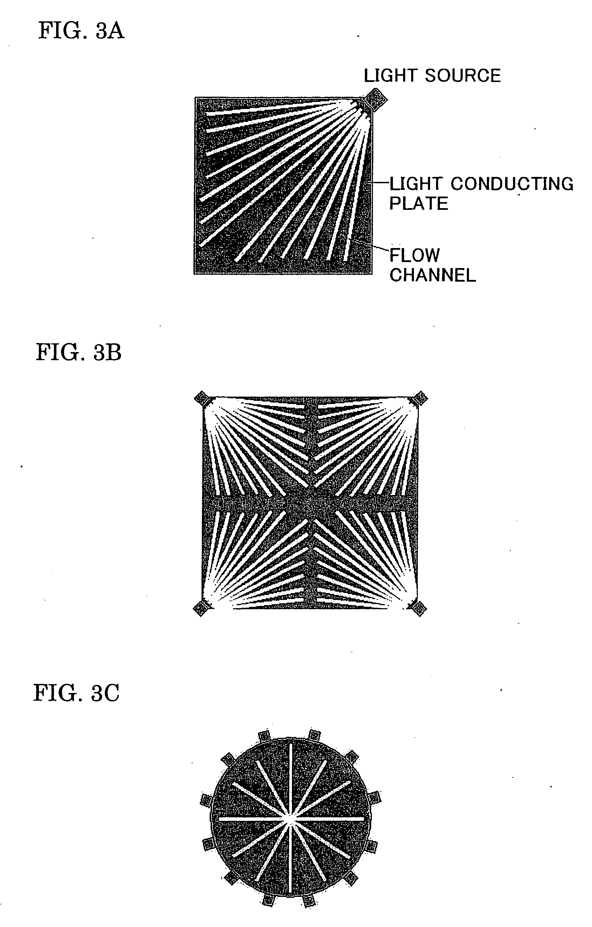

It is preferable that the formation pattern of the through-holes that act as flow channels be formed as a structure that interferes as little as possible with the progress of the light that is emitted from the light source. For example, as is shown in FIGS. 3A and 3B, radial flow channels may be formed that are centered on the light source, or a pattern can be considered in which flow channels are formed in a radial pattern as shown in FIG. 3C, and the light source is disposed so that light is directly delivered from the light source to all of the flow channels. However, these are provided merely by way of example.
Instead of forming through-holes in the light guide plate, it would also be possible to form the light guide plate itself as a porous body, and to form a porous body that has communicating holes. A light guide plate in which through-holes are formed may also be regarded as a type of porous body.

In at least one light emitting surface according to the present invention, i.e., either the front surface or rear surface, a light source is disposed on the side surface, and the surfaces other than the light emitting surface and side surface on which the light source is disposed are light shielding surfaces. In the light guide plate that conducts light to the light emitting surface from the light source, on the front surface part and rear surface pate of the light guide plate, light deflecting elements that cause total reflection can be installed on the surfaces facing each other in order to cause the progression and emission of light in the planar direction in which the front surface part and rear surface part face each other, or a light deflecting element which causes total reflection for the purpose of causing progression and emission of light in the opposite planar direction can be installed on the surface of either the front surface part or rear surface part. In this structure, there may be cases in which the angle of emission from the light guide plate is large in the light that is emitted from the light guide plate (i.e., emission along the surface of the light guide plate); however, there are no particular problems in cases where the photocatalyst is disposed directly above the light emitting surface of the light guide plate.
In cases where the distance between the light guide plate and the photocatalyst sheet is large, a diffusion sheet and a focusing sheet may be disposed on the surface of the light guide plate.
In the present invention, the light guide plate is not limited to a flat plate shape; this plate may also be formed with a curved surface shape, e.g., a cylindrical shape (see FIG. 3C).

Furthermore, in the present invention, light shielding surface refers to the surfaces other than the light emitting surface in the surfaces excluding the side surface on which the light source is disposed. Light shielding surfaces can be formed by forming indentations and projections or coating with a thin film of metal or the like. There are no particular restrictions on the metal used in cases where a thin film coating is formed; any type of metal may be used. Examples of such metals include gold, aluminum, copper and the like.
Furthermore, the side surface on which the light source is disposed may also be coated with a metal except for the portions where light enters.

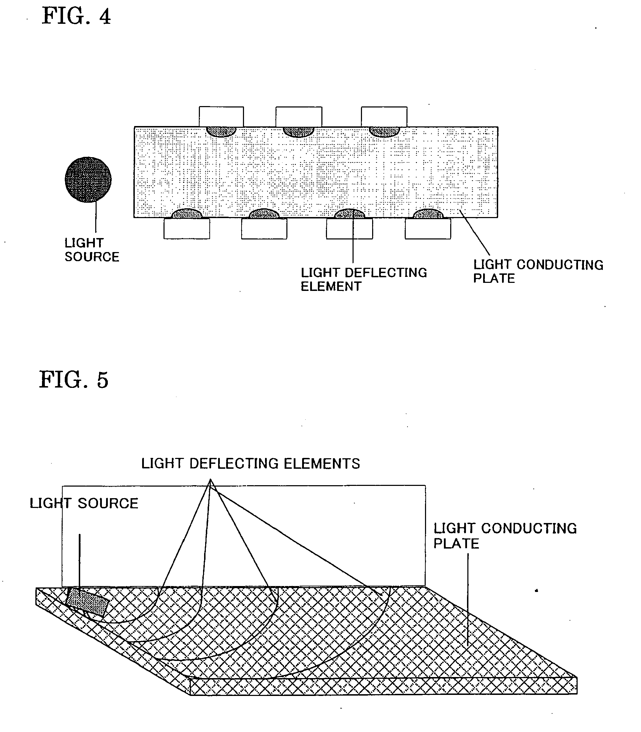

FIG. 4 shows an example in which the light deflecting element of the surface emitting device of the present invention is used. The surface emitting device shown in FIG. 4 is constructed from a light source that supplies light to the light guide plates, and a light guide plate in which light deflecting elements are installed respectively on the front surface part and rear surface part in order to emit light present inside the light guide plate (as a result of the light source) onto the surfaces of both the front surface part and rear surface part. The light deflecting elements comprise convex or concave prisms, dots or the like. Although the light deflecting parts are described as having either a convex or concave circular arc form shape, parts of other shapes such as an elliptical arc, circular cone, cylinder, polygonal cone, polygonal column or the like may be installed at random on the front surface part or rear surface part of the light guide plate. Alternatively, light deflecting elements having convex parts or concave parts with a pillow shape having a circular arc form or polygonal cross section may be continuously or discontinuously disposed. Furthermore, as is likewise in cases where the light deflecting elements have a convex or concave shape such as an elliptical arc, circular cone, cylinder, polygonal cone, polygonal column or the like, an action and effect similar to those of the construction described above can be obtained. A description thereof is superfluous, and is accordingly omitted here.

Furthermore, as is shown in FIG. 5, the light guide plate may also have a construction in which light deflecting elements comprising convex parts or concave parts with a pillow shape having a circular arc form or polygonal cross section are continuously or discontinuously disposed on one or both surfaces of the front surface part and rear surface part. In this example shown in FIG. 5, since the light source is installed in the corner of the light guide plate, the light deflecting elements are installed in concentric positions centered on the light source so as to face the light source. An accommodating part used to accommodate the light source inside the light guide plate is integrally formed on the corner (side surface) of the light guide plate. The light source is inserted into this accommodating part, and is mechanically stabilized.

The light guide plate is formed from a transparent acrylic resin, methacrylic resin (PMMA), polycarbonate (PC) or the like. In the light guide plate, at least one side surface part is formed as an incident part, and the light guide plate has light deflecting elements on the front surface part and/or rear surface part (front surface part and rear surface part in the example shown in FIG. 4).
The transparent acrylic resin, methacrylic resin (PMMA) or polycarbonate resin shows an abrupt drop in transmissivity when the wavelength of the light drops to 400 nm or less, or 350 nm or less. Accordingly, in cases where ultraviolet light having a wavelength of 400 nm or less is used as a light source, a polycarbonate (PC) is desirable. If an amorphous cycloolefin polymer is used, approximately 60% of ultraviolet light having a wavelength of 300 nm is transmitted; accordingly, this is most desirable as a light guide plate in an ultraviolet light emitting device.

A photocatalyst sheet can be used as the photocatalyst that is disposed on the light emitting surface of the light guide plate. The photocatalyst sheet is a sheet in which a photocatalyst is supported on a porous substrate. A foam metal, foam ceramic, resin cloth or the like can be used as the porous substrate. These materials have a high porosity, and are superior in terms of transmission performance. Furthermore, the surface emitting device of the present invention may also have a laminated structure in which light guide plates and photocatalyst sheets are repeatedly laminated.

The surface emitting device of the present invention in which through-holes are formed may also be used as a filter. For example, relatively large particles or the like floating in the air are captured on the surface of the photocatalyst sheet which has a photocatalyst, and small particles are decomposed by the photocatalyst while passing through the photocatalyst sheet. Accordingly, a highly reliable filter can be obtained as the laminated structure of light guide plates and porous bodies is repeated; conversely, however, such a structure has the drawback of a drop in transmission performance.

Furthermore, in the product of the present invention, in cases where ultraviolet light having a peak wavelength of 388 nm or less is emitted, the device acts as an ultraviolet planar light source even if boring is not performed.

In cases where the filter of the present invention is used as the filter of an air cleaner, or as an air conditioner filter or the like, it is desirable that the overall device is thin. As a rule, the thickness of the surface emitting device is 1 mm or less.
A catalytic reaction vessel using the surface emitting device of the present invention has the capacity to decompose organic matter and kill bacteria and the like. The vessel can therefore be used in various fields such as the decomposition and elimination of contaminants in the atmosphere such as NOx, SOx, CO gas, diesel particulates, pollen, dust, mites and the like; the decomposition and elimination of organic compounds in sewage; a light source for killing general bacteria, viruses and the like; the decomposition of harmful gases generated in chemical plants; the decomposition of malodorous components such as acetyl aldehyde, formaldehyde and the like; and a bactericidal light source in devices for manufacturing ultra-pure water. Furthermore, the filter of the present invention can also be used in honeycomb materials for the treatment of automobile exhaust gas, air cleaner or air conditioner filters, sewage filters, various types of water cleaners, elimination of bacteria in hot springs, and insecticides.

### WORKING EXAMPLES

### Working Example 1

### (1) Constituent Members

### 1. Light Conducting Plate: 100 × 100 × 1 mm Size

Light deflecting elements were formed in a concentric pattern by pressing on one side of a methacrylic resin (PMMA), polycarbonate resin (PC) or hydrogenated ring-opened metathesis polymer (HROP: amorphous cycloolefin polymer) sheet.
Through-holes having a diameter of 1mm were formed at a pitch of 1 mm in the light-conducting plate.

### 2. Light Source: LEDs Having Various Peak Emission Wavelengths

Peak emission wavelength 365 nm, output 2.4 mW
Peak emission wavelength 380 nm, output 2.4 mW
Peak emission wavelength 465 nm, output 20 mW
Peak emission wavelength 535 nm, output 20 mW

### (2) Assembly

11 LEDs were attached at intervals of 10 mm to one side of the light guide plate. The light source part was sealed with a moisture-resistant resin. Except for the light source part, the side surface of the light guide plate was covered with Al to a thickness of 0.2 µm by vapor deposition.
The light source was thus disposed on the side surface, and a surface emitting device in which the front surface was a light emitting surface, and the surfaces other than the side surface on which the light source was disposed and the front surface were light shielding surfaces.

### (3) Photocatalyst Sheet

Anatase TiO₂ Mean particle size: 0.03 µm (commercial product)
TiO₂ : S Mean particle size: 0.03 µm
Powdered thiourea (CH₄N₂S) and Ti(OC₃H₇)₄ were mixed in ethanol, and this mixture was concentrated under reduced pressure until a white slurry was obtained. The resulting slurry was fired in ambient air for 2 hr at 588°C to yield a powder. The amount of S doping was set at 2 at% relative to oxygen.
Liquids in which various types of photocatalyst particles were dispersed in alcohol were prepared. A porous fluororesin was immersed and then withdrawn from these liquids, and then heated in ambient air for 0.5 hr at 230°C; the photocatalyst particles were supported on the fluororesin. This procedure was repeated 10 times.

### (4) Evaluation of Photocatalyst Performance

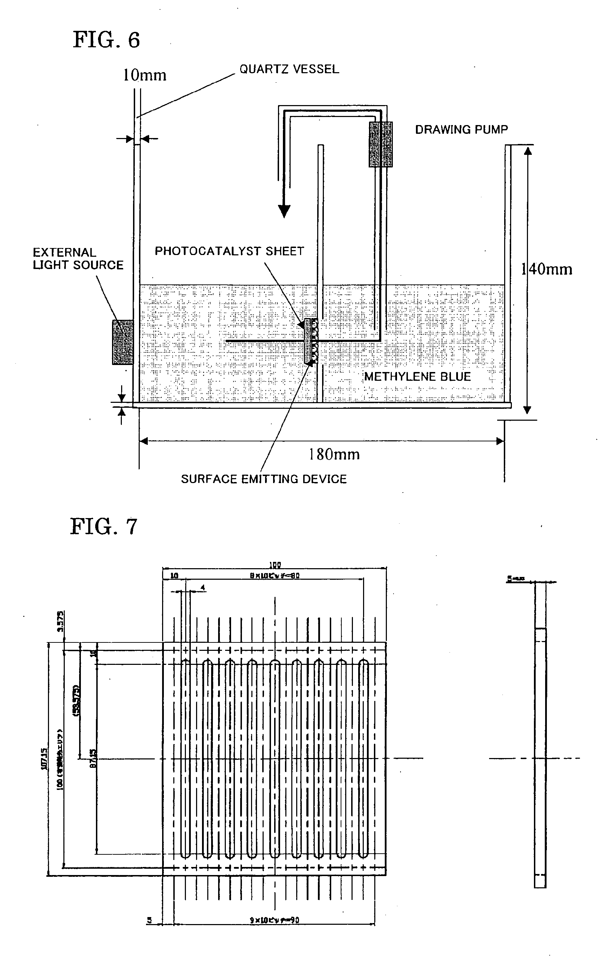

1500 cm³ of a 100 ppm aqueous solution of methylene blue (a type of pigment) was prepared. The photocatalyst sheet was laminated with the light guide plate following the attachment of the LEDs, and a filter was formed.
While the respective LEDs were caused to emit light at 4 V, circulation with filtering by the filter was performed as shown in FIG. 6. The time required for the concentration of the methylene blue solution to reach 90 ppm was measured. The concentration was measured by chemical analysis. Furthermore, the emission intensity of the surface emitting device was measured in ambient air using an illumination meter.
An experiment was also performed in cases where holes were not bored in the surface emitting device. As a comparative example, an experiment was also performed involving the manufacture of a lamp in which 11 LEDs of the same type were disposed, and only the photocatalyst sheet being irradiated from the outside of a quartz glass vessel using this lamp as an external light source. The distance between the light source and the photocatalyst sheet was 90 mm.
The results are shown in Table 1.

**[Table 1]**

| | Light conductor plate material | Area of light plate (cm²) | Holes machined | Type of source | Peak wavelength emitted (nm) | Output (mW) | Quantity | Intensity planar light mW/cm²) | Circulation | Type of photocatalyst | Reaction time (hr) |
|---|---|---|---|---|---|---|---|---|---|---|---|
| 1 | PMMA | 100 | Yes | LED | 365 | 2.4 | 11 | 0.03 | Yes | TiO₂ | 12.2 |
| 2 | PMMA | 100 | Yes | LED | 380 | 2.4 | 11 | 0.07 | Yes | TiO₂ | 7.6 |
| 3 | PMMA | 100 | Yes | LED | 465 | 20 | 11 | 1.33 | Yes | TiO₂:S | 7.7 |
| 4 | PMMA | 100 | Yes | LED | 535 | 20 | 11 | 1.33 | Yes | TiO₂:S | 18.8 |
| 5 | PC | 100 | Yes | LED | 365 | 2.4 | 11 | 0.12 | Yes | TiO₂ | 4.1 |
| 6 | HROP | 100 | Yes | LED | 365 | 2.4 | 11 | 0.17 | Yes | TiO₂ | 3.1 |
| 7 | PC | 100 | No | LED | 365 | 2.4 | 11 | 0.12 | No | TiO₂ | 26.7 |
| 8* | None | None | No | External | 365 | 2.4 | 11 | not measured | Yes | TiO₂ | >50 |

| | | | | | | | | | | | |
|---|---|---|---|---|---|---|---|---|---|---|---|
| * Comparative example | | | | | | | | | | | |

The reaction time was shorter as the emission wavelength became shorter. The reaction time in the case of an external light source was long. This was presumably because the light was absorbed in the solution and tended not to reach the photocatalyst. The reaction time was shortened by circulation. This was presumably because the catalyst and methylene blue were efficiently kept in contact using circulation.

### Working Example 2

### (1) Constituent Members

### 1. Light Conducting Plate: 100 × 100 5 × mm Size

Light deflecting elements were formed by pressing on one side of a Derpet (560 MF, PMMA) sheet manufactured by Asahi Kasei having the shape shown in FIG. 7. The light deflecting elements were designed so that both surfaces of the light guide plate emitted light. Slit-form through-holes having the shape shown in FIG. 7 were formed in this light guide plate by mechanical working.

### 2. Light Source

(LEDs Having Various Emission Peak Wavelengths)
Peak wavelength 365 nm, output 2.4 mW
Peak wavelength 410 nm, output 7.4 mW

### (External Light Source)

Black light with peak wavelength of 365 nm, output of 7 W

### (2) Assembly

On two side surfaces of the light guide plate in the direction of the long axis of the slit-form through-holes, 10 LEDs were attached to the upper side of one side surface, and 10 LEDs were attached to the lower side of the other side surface, so a total of 20 LEDs were attached. In this case, the LEDs were disposed so as to face the light guide plate between the slit-shaped through-holes. The light source part was sealed with a moisture-resistant resin. Except for the light source part, the side surface of the light guide plate was covered with Al by vapor deposition to a thickness of 0.2 µm.
A surface emitting device was obtained in which the light source was thus disposed on the side surface, the front surface and rear surface were light emitting surfaces, and the surfaces other than the side surface on which the light source was disposed, the front surface, and the rear surface were light shielding surfaces.

### (3) Photocatalyst Sheet

Anatase TiO₂ Mean particle size: 0.03 µm (MT-600; Teika)
Rutile TiO₂ Mean particle size: 0.03 µm (MPT-623; Ishihara Sangyo)
Liquids were prepared in which various types of photocatalyst particles were dispersed in alcohol. A porous fluororesin was immersed in the liquids, withdrawn therefrom, and dried at room temperature to form a photocatalyst sheet.

### (4) Evaluation of Photocatalyst Performance

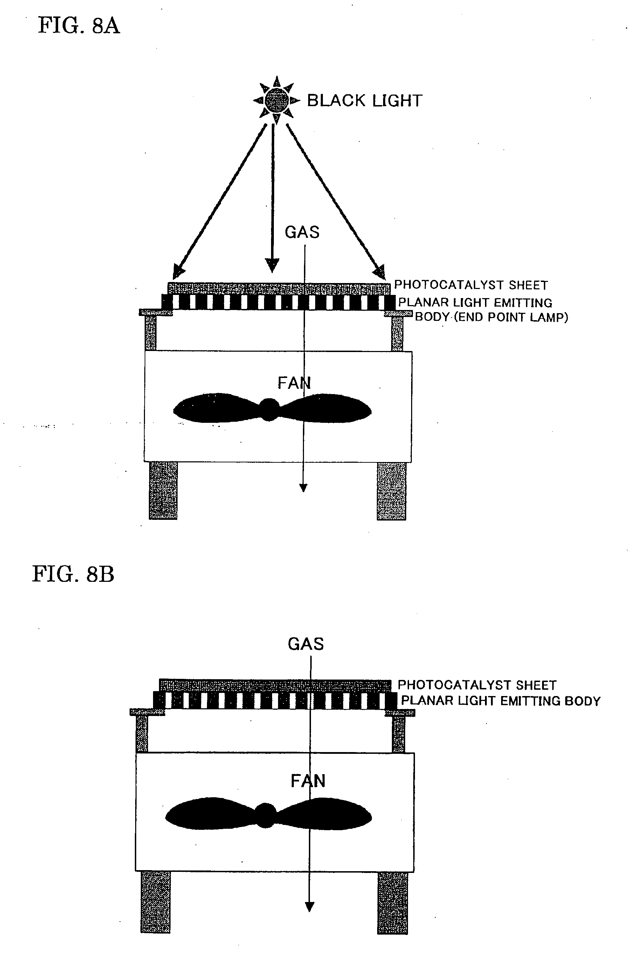

One or two surface emitting devices were disposed in a desiccator made of quartz glass. Using a micro-syringe, acetaldehyde gas was injected into the desiccator until the concentration reached 20 ppm. Measurement of the concentration was performed using a multi-gas monitor.
Photocatalyst sheets were caused to adhere tightly to the front surface (upper surface) or front and rear surfaces (upper and lower surfaces) of the light guide plate.
While the respective LEDs were caused to emit light at 4 V, the gas was circulated and passed through the photocatalyst sheet as shown in FIG. 8B. The time required for the concentration of the acetaldehyde gas to drop to 10 ppm was measured, and the mean decomposition rate was calculated from these results. The emission intensity of the light guide plate was measured beforehand using an illumination meter without a photocatalyst sheet.

As a comparative example, the decomposition rate was measured at various illuminations using a black light as an external light source as shown in FIG. 8A.
The results are shown in Table 2.

**[Table 2]**

| | Light conductor plate material | Area of light conductor plate (cm²) | Type of light source | Number of surface emitting plates | Peak wavelength of emitted light (nm) | Rated output (mw) | Quantity | Current (mA) | Intensity of surface emitted light (mW/cm²) | Type of photocatalyst | Placement of photocatalyst sheet (surface) | Decomposition rate (ppm/min) |
|---|---|---|---|---|---|---|---|---|---|---|---|---|
| 10 | Derpet | 100 | LED | 1 | 365 | 2.4 | 20 | 40 | 0.150 | Rutile | Top | 0.073 |
| 11 | Derpet | 100 | LED | 1 | 410 | 7.4 | 20 | 30 | 0.160 | Rutile | Top | 0.051 |
| 12* | Derpet | 100 | External | none | 365 | none | none | none | 0.151 | Rutile | Top | 0.032 |
| | | | | | | | | | | | | |
| 13 | Derpet | 100 | LED | 1 | 365 | 2.4 | 20 | 80 | 0.339 | Rutile | Top | 0.161 |
| 14 | Derpet | 100 | LED | 1 | 365 | 2.4 | 20 | 80 | 0.339 | Rutile | Top/bottom | 0.328 |
| 15 | Derpet | 100 | LED | 2 | 365 | 2.4 | 20 | 80 | 0.339 | Rutile | Top/bottom | 0.582 |
| 16 | Derpet | 100 | LED | 1 | 410 | 7.4 | 20 | 50 | 0.430 | Rutile | Top | 0.111 |
| 17 | Derpet | 100 | LED | 1 | 410 | 7.4 | 20 | 50 | 0.430 | Rutile | Top/bottom | 0.201 |
| 18 | Derpet | 100 | LED | 2 | 410 | 7.4 | 20 | 50 | 0.430 | Rutile | Top/bottom | 0.382 |
| 19* | Derpet | 100 | External | none | 365 | none | none | none | 0.355 | Rutile | Top | 0.08 |
| | | | | | | | | | | | | |
| 20 | Derpet | 100 | LED | 1 | 365 | 2.4 | 20 | 80 | 0.398 | Anatase | Top | 0.151 |
| 21 | Derpet | 100 | LED | 1 | 410 | 7.4 | 20 | 50 | 0.429 | Anatase | Top | 0.098 |
| 22* | Derpet | 100 | External | none | 365 | none | none | none | 0.355 | Anatase | Top | 0.071 |

| | | | | | | | | | | | | |
|---|---|---|---|---|---|---|---|---|---|---|---|---|
| * Comparative examples | | | | | | | | | | | | |

It is seen that the rutile photocatalyst had an activity comparable to that of the anatase photocatalyst. In cases where the surface emitting device of the present invention was used, the photocatalytic performance was discovered to be higher than that when an external light source was used.
This is presumably because the light guide plate and photocatalyst sheet are caused to adhere tightly to each other, for which reason all of the light emitted from the light guide plate contributes to the catalytic reaction. On the other hand, in cases where an external light source is used, it appears that a portion of the emitted light does not contribute to the photocatalytic reaction, so that the efficiency is poor.
In cases where photocatalyst sheets are disposed on the upper and lower surfaces of the light guide plate, it is seen that the photocatalytic performance increases. Furthermore, in cases where light guide plates are laminated, it was found that the photocatalytic performance increased even further.
Thus, in cases where light guide plates are used, it was found that the special feature of the disposition of a photocatalyst with a large surface area in an extremely thin space can be accomplished by alternately laminating a plurality of photocatalysts and a plurality of light guide plates, and that the present invention is optimally suitable for use in air cleaning devices in thin compact spaces.

### INDUSTRIAL APPLICABILITY

The surface emitting device of the present invention can be used in various fields such as the decomposition and elimination of contaminants in the atmosphere such as NOx, SOx, CO gas, diesel particulates, pollen, dust, mites and the like; the decomposition and elimination of organic compounds in sewage; a light source for killing general bacteria, viruses and the like; the decomposition of harmful gases generated in chemical plants; and the decomposition of bad-smelling components. The present invention may be developed for use as any type of filter in the fields described above, and can also be used in air cleaning, sewage filtration, various types of water cleaners, insecticides and other such applications.
In addition, the present invention can also be used as a light source for fixing the toner used in digital photo-printers for digital cameras, light sources used to cure ultraviolet-curable resins, and the like. Furthermore, using the characteristic that insects tend to be drawn to ultraviolet light centering on a wavelength of 365 nm, the present invention can also be used as a sheet-shaped insect capturing panel, and is useful for the prevention of malaria.

## Claims

1. A surface emitting device, comprising:
a light source; and
a light guide plate with the light source disposed on a side surface thereof, wherein
at least one of the front surface and rear surface of the light guide plate is a light emitting surface for emitting light from the light source,
surfaces other than the light emitting surface of the light guide plate and the side surface on which the light source is disposed are formed as light shielding surfaces, and
the emitted light has a peak wavelength of 388 nm or less.

2. A surface emitting device, comprising:
a light source; and
a light guide plate with the light source disposed on a side surface thereof, wherein
at least one of front and rear surfaces of the light guide plate is a light emitting surface for emitting light from the light source,
surfaces other than the light emitting surface of the light guide plate and the side surface on which the light source is disposed are formed as light shielding surfaces, and
the light guide plate is formed as a porous body.

3. The surface emitting device according to Claim 2, wherein the light guide plate formed as a porous body has through-holes for communicating between the front surface and rear surface.

4. The surface emitting device according to any one of Claims 1 through 3, wherein the light guide plate has a flat plate shape or curved surface shape.

5. The surface emitting device according to Claim 3, wherein the surface emitting device has a plurality of light guide plates of predetermined shape disposed with a predetermined gap left between the plates, the gaps constituting through-holes.

6. The surface emitting device according to any one of Claims 2 through 5, wherein the surface emitting device emits light having a peak wavelength of 540 nm or less.

7. The surface emitting device according to Claim 6, wherein the surface emitting device emits light having a peak wavelength of 420 nm or less.

8. The surface emitting device according to Claim 7, wherein the surface emitting device emits light having a peak wavelength of 388 nm or less.

9. The surface emitting device according to any one of Claims 1 through 8, wherein the surface emitting device emits light having a peak wavelength within a range of 355 to 375 nm.

10. The surface emitting device according to any one of Claims 1 through 9, wherein the light source is an LED.

11. The surface emitting device according to any one of Claims 1 through 9, wherein the light source is an inorganic EL.

12. The surface emitting device according to any one of Claims 1 through 9, wherein the light source is a cold cathode fluorescent lamp.

13. The surface emitting device according to any one of Claims 1 through 12, wherein a photocatalyst or a porous body supporting a photocatalyst is disposed on the light emitting surface of the light guide plate.

14. The light emitting device according to Claim 13, wherein a distance between the light guide plate and the photocatalyst or porous body supporting this photocatalyst is zero.

15. The surface emitting device according to Claim 13, wherein at least one of a diffusion sheet and a focusing sheet is disposed between the light emitting surface of the light guide plate and the photocatalyst or porous body supporting the photocatalyst.

16. The surface emitting device according to any one of 13 through 15, wherein the photocatalyst is a rutile titanium oxide.

17. A surface emitting device, comprising:
a light source; and
a light guide plate with the light source disposed on a side surface thereof, wherein
a plurality of through-holes that communicate between a front and rear surface are provided so that a fluid can pass through this light guide plate, and
the surface in which these through-holes are formed is a light emitting surface for emitting light from the light source.

18. The surface emitting device according to Claim 17, wherein surfaces other than the light emitting surface and the side surface on which the light source is disposed are formed as light shielding surfaces.

19. A filter in which the surface emitting device according to any one of Claims 2 through 18 is used.

20. An air cleaner or air conditioner filter in which the filter according to Claim 19 is used.

21. A liquid cleaning filter in which the filter according to Claim 19 is used.

## Amended claims

### Amended claims under Art. 19.1 PCT

**1.** A surface emitting device comprising:
a light source; and
a light guide plate in which the light source is disposed on a side surface, wherein
a plurality of through-holes which communicate between a front surface and a rear surface are formed so that a fluid can pass through the light guide plate,
a surface in which these through-holes are formed is a light emitting surface for emitting light from the light source, and surfaces other than this light emitting surface and the side surface on which the light source is disposed are formed as light shielding surfaces.

**2.** The surface emitting device according to claim 1, wherein a photocatalyst or a porous body on which a photocatalyst is supported is disposed on at least one of the front and rear surfaces of the light guide plate.

**3.** The surface emitting device according to claim 1, wherein a photocatalyst or porous body on which a photocatalyst is supported is disposed so as to adhere tightly to the light guide plate.

**4.** The surface emitting device according to claim 3, wherein a photocatalyst or porous body on which a photocatalyst is supported is disposed so as to adhere tightly to through-holes in the light guide plate.

**5.** The surface emitting device according to any one of claims 1 through 4, wherein the surface emitting device emits light having a peak wavelength of 540 nm or less.

**6.** The surface emitting device according to claim 5, wherein the surface emitting device emits light having a peak wavelength of 420 nm or less.

**7.** The surface emitting device according to claim 6, wherein the surface emitting device emits light having a peak wavelength of 388 nm or less.

**8.** The surface emitting device according to claim 7, wherein the surface emitting device emits light having a peak wavelength within a range of 355 to 375 nm.

**9.** A filter using the surface emitting device according to any one of claims 1 through 8.

**10.** An air cleaner or air conditioner filter in which the filter according to claim 9 is used.

**11.** A liquid cleaning filter in which the filter according to claim 9 is used.
